(19) Europäisches Patentamt
European Patent Office
Office européen des brevets

(11) Veröffentlichungsnummer: **0 042 181**
**A2**

(12) **EUROPÄISCHE PATENTANMELDUNG**

(21) Anmeldenummer: **81105610.0**

(22) Anmeldetag: **12.09.79**

(51) Int. Cl.³: **C 07 C 143/38**, C 07 C 143/40, C 07 C 143/68

(30) Priorität: **23.09.78 DE 2841519**

(43) Veröffentlichungstag der Anmeldung: **23.12.81 Patentblatt 81/51**

(84) Benannte Vertragsstaaten: **CH DE FR GB**

(60) Veröffentlichungsnummer der früheren Anmeldung nach Art. 76 EPÜ: **0009679**

(71) Anmelder: **BAYER AG, Zentralbereich Patente, Marken und Lizenzen, D-5090 Leverkusen 1, Bayerwerk (DE)**

(72) Erfinder: **Schieder, Rudolf, Dr., Deutscher Ring 6, D-5030 Hürth (DE)**
Erfinder: **Telle, Helmut, Brabanter Strasse 17, D-5000 Köln 1 (DE)**
Erfinder: **Raue, Roderich, Dr., Berta-von-Suttner-Strasse 48, D-5090 Leverkusen (DE)**
Erfinder: **Brinkwerth, Wolfgang, Dr., Sanderstrasse 89a, D-5060 Bergisch-Gladbach 2 (DE)**

(54) **Diphenylderivate.**

(57) Diphenylderivate der Formel

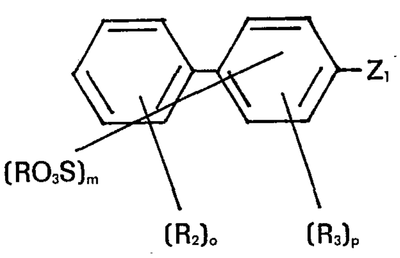

(I)

in der

R für Wasserstoff, ein salzbildendes Kation, einen Alkylrest mit 1 bis 8 Kohlenstoffatomen oder einen gegebenenfalls durch nicht chromophore Gruppen substituierten Aralkylrest,

$R_2$, $R_3$ unabhängig voneinander für Wasserstoff, Alkyl, Trifluormethyl, Alkoxy, Aralkoxy, Alkenyloxy, Halogen, die Carboxyl-, Cyan-, Alkylsulfon-, Arylsulfon-, Carbonamid-, Sulfonamid- oder die Carbonsäureestergruppe stehen,

m 1 oder 2 bedeutet,

o, p unabhängig voneinander für 0, 1 oder 2 stehen und

$Z_1$ die Aldehydgruppe oder einen Rest der Formeln

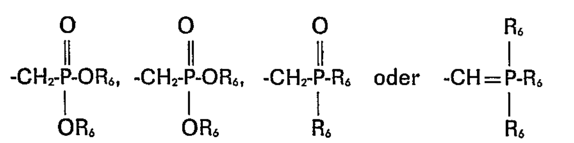

mit $R_6$ = $C_1$-$C_6$-Alkyl, Aryl, Aralkyl, Cycloalkyl, bezeichnet.

EP 0 042 181 A2

0042181

BAYER AKTIENGESELLSCHAFT 5090 Leverkusen, Bayerwerk

Zentralbereich
Patente, Marken und Lizenzen PG/m-c

Diphenylderivate

Die Erfindung betrifft Diphenylderivate der Formel

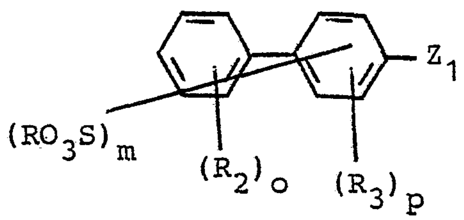

(I)

in der

R für Wasserstoff, ein salzbildendes Kation, einen Alkylrest mit 1 bis 8 Kohlenstoffatomen oder einen gegebenenfalls durch nicht chromophore Gruppen substituierten Aralkylrest,

$R_2$ $R_3$ unabhängig voneinander für Wasserstoff, Alkyl, Trifluormethyl, Alkoxy, Aralkoxy, Alkenyloxy, Halogen, die Carboxyl-, Cyan-, Alkylsulfon-, Arylsulfon-, Carbonamid-, Sulfonamid- oder die Carbonsäureestergruppe stehen,

m 1 oder 2 bedeutet,

o, p unabhängig voneinander für 0, 1 oder 2 stehen, und

$Z_1$ die Aldehydgruppe oder einen Rest der Formeln

$$-CH_2-\overset{O}{\underset{OR_6}{P}}-OR_6,\quad -CH_2-\overset{O}{\underset{OR_6}{P}}-OR_6,\quad -CH_2-\overset{O}{\underset{R^6}{P}}-R_6 \text{ oder } -CH{=}\overset{R_6}{\underset{R_6}{P}}-R_6$$

mit $R_6 = C_1-C_6$-Alkyl, Aryl, Aralkyl, Cycloalkyl, bezeichnet.

Als Substituenten seien beispielsweise genannt:

$C_1$- bis $C_5$-Alkylreste, die durch Hydroxy, Cyan, Halogen oder Phenyl weiter substituiert sein können, wie Methyl, Ethyl, Cyanethyl, tert. Butyl; Benzyl; Halogenatome, wie Chlor, Brom oder Fluor, vorzugsweise Chlor; $C_1$ bis $C_5$-Alkoxyreste, wie Methoxy, Ethoxy, Butoxy und Isopropoxy; Allyloxy; Benzyloxy; gegebenenfalls durch Hydroxy substituierte $C_1$ bis $C_5$-Alkylsulfonylreste, wie Methylsulfonyl, Ethylsulfonyl, n-Butylsulfonyl, ß-Hydroxyethylsulfonyl; der Benzylsulfonylrest; der Phenylsulfonylrest; gegebenenfalls durch $C_1$- bis $C_4$-Alkylreste mono- oder disubstituierte Carbonamid- oder Sulfonamidgruppen; sowie Carbonsäure-$C_1$- bis $C_4$-alkylestergruppen.

Als salzbildende Kationen kommen ein- oder zweiwertige Metalle, wie Natrium, Kalium, Lithium, Magnesium, Calcium, Barium, Mangan und Zink in Frage; sowie Ammoniumsalze und deren Substitutionsprodukte, die man durch

Umsetzung der zugrunde liegenden Säuren mit Mono-, Di- und Trimethylamin, Mono-, Di- und Triethylamin, Mono-, Di- und Triethanolamin, Methyldiethanolamin, Ethyldiethanolamin, Dimethylethanolamin, Diethylethanolamin, Mono-, Di- und Triisopropanolamin, Methyldiisopropanolamin, Ethyldiisopropanolamin, Dimethylisopropanolamin, n-Butylamin, sek. Butylamin, Dibutylamin, Diisobutylamin, Triethoxyethanolamin, Pyridin, Morpholin oder Piperidin erhält.

Die erfindungsgemäßen Diphenylderivate dienen als Ausgangsprodukte zur Herstellung von Stilbenverbindungen, die ihrerseits als Weißtöner und Laserfarbstoffe Verwendung finden. Dabei kondensiert man eine Verbindung der Formel (I), in der $Z_1$, die Aldehydgruppe bezeichnet, mit einer Verbindung der Formel (I), in der $Z_1$ einen der angegebenen P-haltigen Reste bezeichnet, in Gegenwart einer stark basischen Alkaliverbindung und in Gegenwart eines vorzugsweise hydrophilen stark polaren Lösungsmittels.

Geeignete Lösungsmittel sind beispielsweise Toluol; Xylol; Chlorbenzol; Alkohohle, wie Ethanol; Ethylenglykolmonomethylether; vorzugsweise jedoch N-Methylpyrrolidon; Dimethylformamid; Diethylformamid; Dimethylacetamid oder Dimethylsulfoxid.

Die Temperatur, bei welcher die Umsetzung durchgeführt wird, kann in weiten Grenzen schwanken. Sie wird bestimmt:

a) durch die Beständigkeit des verwendeten Lösungsmittels gegenüber den Reaktionsteilnehmern, insbesondere gegenüber den stark basischen Alkaliverbindungen,

b) durch die Reaktivität der Kondensationspartner und

c) durch die Wirksamkeit der Kombination Lösungsmittel/Base als Kondensationsmittel.

Vorzugsweise liegt sie etwa im Bereich von 30°C bis 60°C, doch können in vielen Fällen schon befriedigende Resultate bei Raumtemperatur (ca. 20°C) einerseits oder andererseits bei Temperaturen von 100°C, ja sogar bei der Siedetemperatur des Lösungsmittels erreicht werden, wenn dies aus Gründen der Zeitersparnis oder dadurch erwünscht ist, ein weniger aktives, aber billigeres Kondensationsmittel einzusetzen. Grundsätzlich sind somit auch Reaktionstemperaturen von 10 bis 180°C möglich.

Als stark basische Alkaliverbindungen kommen vor allem die Hydroxide, Amide und Alkoholate (vorzugsweise solche primärer 1 bis 4 Kohlenstoffatome enthaltender Alkohole) der Alkalimetalle in Betracht, wobei aus ökonomischen Gründen solche des Lithiums, Natriums und Kaliums von vorwiegendem Intersse sind. Es können jedoch grundsätzlich und in besonderen Fällen auch Alkalisulfide und -carbonate, Arylalkaliverbindungen, wie Phenyl-Lithium oder stark basische Amine (einschließlich Ammoniumbasen, z.B. Trialkylammoniumhydroxide) mit Erfolg verwendet werden.

Die als Ausgangsstoffe benötigten Phosphorverbindungen der Formel I werden in an sich bekannter Weise erhalten, indem man Halogenmethylverbindungen, vorzugsweise Chlormethyl- oder Brommethylverbindungen der Formel

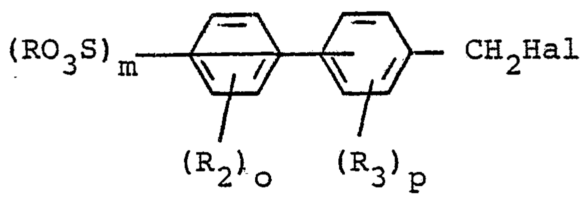

(II)

in denen

die Reste R, $R_2$, $R_3$ und die Indizes m, o und p die gleiche Bedeutung wie in Formel I haben und

Hal für Chlor oder Brom steht,

mit Phosphorverbindungen der Formeln

$(R_6O)_3P$, $R_6P(OR_6)_2$, $(R_6)_2POR_6$, $P(R_6)_3$,

in denen

$R_6$ die oben angegebene Bedeutung hat, und wobei an Sauerstoff gebundene Reste $R_6$ vorzugsweise niedrige Alkylgruppen, unmittelbar an Phosphor gebundene Reste $R_6$ dagegen vorzugsweise Arylreste sind,

umsetzt.

Die Halogenmethylverbindungen der Formel II lassen sich aus der entsprechenden Methylverbindung gemäß der in der DE-PS 234 913 beschriebenen Arbeitsweise durch Umsetzung mit Phosphorpentachlorid und Chlor und anschließende Verseifung der Sulfochloride herstellen oder durch Bromierung mit N-Bromsuccinid gemäß den Angaben der DE-OS 2 262 340. Die Brommethylgruppe läßt sich auch durch Umsetzung des entsprechenden Diphenylderivates mit Paraformaldehyd und Natriumbromid in Schwefelsäure-Eisessig-Mischung nach der in der DE-OS 2 262 340 beschriebenen Arbeitsweise einführen. Ein weiterer Weg zur Herstellung der Halogenmethylverbindungen der Formel II besteht in der Reduktion der Biphenylaldehydsulfonsäuren mit Natriumborhydrid zu den Hydroxymethylbiphenylsulfonsäuren, der Überführung in die Halogenmethylbiphenylsulfochloride mit Thionylchlorid oder Phosphorpentachlorid und der anschließenden Veresterung der Sulfochloride zu den Sulfonsäureestern.

Geeignete Halogenmethylverbindungen der Formel II sind: 4-Chlormethylbiphenyl-4'-sulfonsäureethylester, 4-Brommethylbiphenyl-4'-sulfonsäuremethylester, 4-Chlormethylbiphenyl-3-sulfonsäureethylester, 4-Chlormethylbiphenyl-3,4'-disulfonsäurediethylester, 4-Brommethyl-4'-methylbiphenyl-3,3'-disulfonsäurediethylester, 4-Brommethyl-4'-brombiphenyl, 4-Brommethyl-4'-phenylsulfonylbiphenyl, 4-Brommethyl-4'-cyanbiphenyl, 4-Brommethyl-3'-methyl-4'-brombiphenyl.

Die Aldehyde der Formel I ($Z_1$ bzw. $Z_2$ = CHO) lassen sich aus den Halogenmethylverbindungen durch Umsetzung mit Hexamethylentetramin in Essigsäure (Sommelet-Reaktion) herstellen. Man erhält sich auch durch Nachsulfierung von Biphenylaldehyden mit Oleum gemäß der in der DE-OS 2 525 681 beschriebenen Arbeitsweise. Ein weiterer Weg zu den Biphenylaldehydsulfonsäuren besteht in der Umsetzung von halogenierten Biphenylaldehyden mit Natriumsulfit.

Geeignete Biphenylaldehyde der Formel I sind: Biphenyl-4-aldehyd-4'-sulfonsäure, Biphenyl-4-aldehyd-3-sulfonsäure, Biphenyl-4-aldehyd-3,4'-disulfonsäure, Biphenyl-4-aldehyd, 3,4-Dichlorbiphenyl-4'-aldehyd, 4-Methylbiphenyl-4'-aldehyd-3,3'-disulfonsäure, 3-Chlorbiphenyl-4'-aldehyd-4-sulfonsäure, 4-Brombiphenyl-4'-aldehyd, 4-Cyanbiphenyl-4'-aldehyd, 3-Methyl-4-brombiphenyl-4'-aldehyd.

Beispiel 1

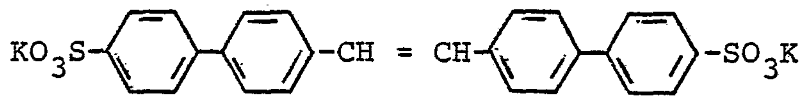

In einem Reaktionsgefäß werden unter Ausschluß von Feuchtigkeit und Luftsauerstoff 40,0 g Kalium-tert.-butylat (ca. 90%ig) in 300 ml Dimethylformamid (wasserfrei) suspendiert. Bei einer Reaktionstemperatur von 40-50°C wird innerhalb von 1 Std. eine filtrierte Lösung aus 40 g biphenyl-4-aldehyd-4'-sulfonsaurem Kalium (ca. 80%ig) in 1.100 ml Dimethylformamid, die mit einer Lösung aus 68 g 4-Diethoxyphosphonmethylbiphenyl-4'-sulfonsäureethylester in 100 ml Dimethylformamid verrührt wurde, zugetropft. Es wird 2 Stdn. bei 40-50°C gerührt, bis im Dünnschichtchromatogramm keine der Ausgangsverbindungen nachgewiesen werden kann. Unter Zusatz von 300 ml Wasser wird ca. 2 Stdn. bis zur Entfärbung der Reaktionsmischung auf 80-90°C erhitzt. Nach Abkühlen, Absaugen, Waschen mit wenig Wasser und Trocknen im Vakuum bei 80°C wird die Verbindung der oben angegebenen Formel als farbloses kristallines Produkt erhalten. Ausbeute: 56,8 g = 62,5 % der Theorie.

Zur Überführung in die leichter löslichen Ammoniumsalze erhitzt man das Kaliumsalz mit halbkonzentrierter Ammoniaklösung, mit Triethanolamin oder mit Tris-2-hydroxyethoxyethylamin.

Das als Ausgangsmaterial verwendete biphenyl-4-aldehyd-4'-sulfonsaure Kalium wurde in folgender Weise gewonnen:

182 g Biphenyl-4-aldehyd wurden portionsweise in 400 g 20%ig. Oleium so eingetragen, daß die Temperatur langsam von 25°C auf 50°C ansteigt. Die Temperatur wird 3 Stdn. bei 50°C gehalten und die Reaktionsmischung dann auf 1.000 g Eis und 30 g Kaliumchlorid ausgetragen. Das biphenyl-4-aldehyd-4'-sulfonsaure Kalium fällt als flockiger Niederschlag aus. Das Rohprodukt wird unter Zusatz von A-Kohle aus Wasser umkristallisiert. Ausbeute; 258,3 g = 85 % der Theorie.

Der als Ausgangsmaterial verwendete 4-Diethoxyphosphonmethylbiphenyl-4'-sulfonsäureethylester wurde in folgender Weise hergestellt:

1. Stufe: 4-Hydroxymethylbiphenyl-4'-sulfonsäure

166 g biphenyl-4-aldehyd-4'-sulfonsaures Kalium (80%ig) werden in 600 ml Wasser suspendiert und mit 100 ml 10%iger Natronlauge versetzt. Unter Stickstoff wird eine Lösung aus 6,2 g Natriumborhydrid in 300 ml Wasser und 30 ml 10%iger Natronlauge portionsweise zugesetzt. Unter starker Wasserstoffentwicklung steigt die Temperatur auf 30°C. Es wird 14 Stdn. bei Raumtemperatur gerührt. Nach vollständiger Reduktion wird vorsichtig mit konz. Salzsäure angesäuert und die Suspension zur Zerstörung des Borkomplexes und zur Bildung der Säure 3 Stdn.

auf 80°C erhitzt. Nach Abkühlen saugt man die 4-Hydroxymethylbiphenyl-4'-sulfonsäure ab, wäscht sie mit wenig Wasser und trocknet sie im Vakuum bei 100°C.
Ausbeute: 113 g = 93 % der Theorie.

<u>2. Stufe:</u> 4-Chlormethylbiphenyl-4'-sulfonsäurechlorid

113 g 4-Hydroxymethylbiphenyl-4'-sulfonsäure werden in 800 ml Chlorbenzol suspendiert und zur vollständigen Entfernung von Wasser 250 ml Chlorbenzol abdestilliert. Dann wird auf 80-90°C abgekühlt und nach Zusatz von 3 ml Dimethylformamid und 1 g Phosphorpentachlorid werden innerhalb von 2 Stdn. 150 ml Thionylchlorid zugetropft. Die Temperatur wird 14 Stdn. bei 85-90°C gehalten. Das Lösungsmittel und überschüssiges Thionylchlorid werden im Vakuum fast bis zur Trockne abdestilliert. Der Rückstand wird mit 900 ml Methylcyclohexan heiß extrahiert, wobei Salze und nicht umgesetzte Sulfonsäure als Rückstand verbleiben, während das Sulfochlorid sich aus Methylcyclohexan beim Abkühlen als farblose kristalline Verbindung abscheidet.
Ausbeute: 100,6 g = 78 % der Theorie,
Schmelzpunkt: 110-112°C.

<u>3. Stufe:</u> 4-Chlormethylbiphenyl-4'-sulfonsäureethylester

98 g 4-Chlormethylbiphenyl-4'-sulfonsäurechlorid werden unter Feuchtigkeits- und Sauerstoffausschluß in 400 ml Tetrahydrofuran (wasserfrei) gelöst und mit einem

10%igen Überschuß an Natriumalkoholat, hergestellt aus 8 g Natrium und 1.000 ml absoluten Alkohol, portionsweise versetzt, so daß die Reaktionstemperatur unter Eiskühlung bei 10-15°C gehalten wird. Nach einstündigem Rühren bei Raumtemperatur wird das Lösungsmittelgemisch im Vakuum bei 50°C abgezogen. Der Rückstand wird mit 1.200 ml Di-sek.-butylether (wasserfrei) heiß extrahiert. Nach Abkühlen, Absaugen und Trocknen im Vakuum bei 50°C erhält man 68,5 g 4-Chlormethylbiphenyl-4'-sulfonsäure-ethylester.
Ausbeute: 68 % der Theorie,
Schmelzpunkt: 112 - 116°C.

<u>4. Stufe:</u> 4-Diethoxyphosphonmethylbiphenyl-4'-sulfon-säureethylester

48,5 g 4-Chlormethylbiphenyl-4'-sulfonsäureethylester werden unter Feuchtigkeits- und Sauerstoffausschluß in 100 ml wasserfreiem Xylol suspendiert. Innerhalb von 2 Stdn. werden bei 130-140°C beginnend 75 g Triethylphosphit unter gleichzeitiger Abdestillation des entstehenden Ethylchlorids und von Xylol und Triethylphosphit zugetropft, so daß die Innentemperatur langsam auf 165-170°C ansteigt. Die Reaktionsmischung wird 7 Stdn. bei dieser Temperatur gehalten. Überschüssiges Triethylphosphit wird bei 160° und 20 mbar abdestilliert. Das als Rückstand verbleibende Rohöl (68 g) wird in 100 ml Dimethylformamid aufgenommen und in dieser Form mit biphenyl-4-aldehyd-4'-sulfonsaurem Kalium umgesetzt.

Beispiel 2

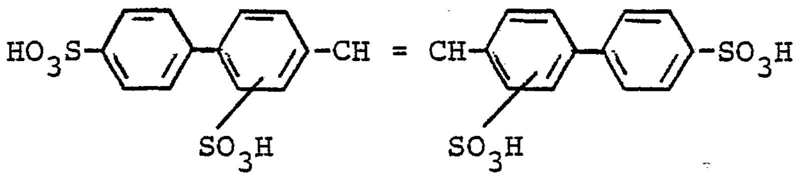

10 g der gemäß Beispiel 1 hergestellten Verbindung werden in 50 ml 20%ig. Oleum bei 0-10°C eingetragen und 30 Min. bei 5°C verrührt. Innerhalb von 1 Std. wird die Temperatur auf 25°C erhöht. Nach Austragen auf ein Gemisch von Eis und Wasser wird mit Calciumhydroxid neutralisiert, das entstehende Calciumsulfat abfiltriert und gründlich mit Wasser gewaschen. Das Filtrat wird mit A-Kohle geklärt und das Calciumsalz an einem stark sauren Ionenaustauscher in die freie Sulfonsäure überführt.

In ähnlicher Weise wie in den vorstehenden Beispielen beschrieben, können die in der nachfolgenden Tabelle aufgeführten Diphenylstilbensulfonsäuren der Formel

A - CH = CH - A'

dargestellt werden.

| A | A' |
| --- | --- |
| 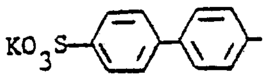 | 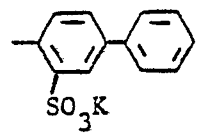 |
| 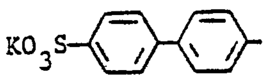 | 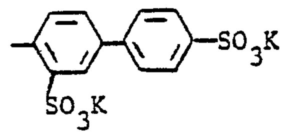 |
| 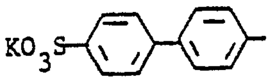 | 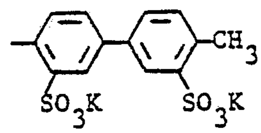 |
| 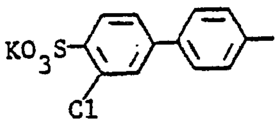 | 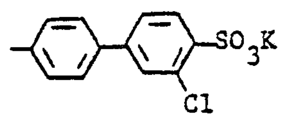 |
| 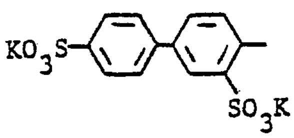 | 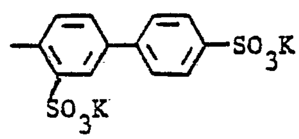 |
| 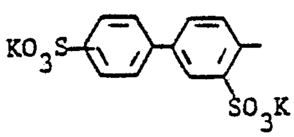 | 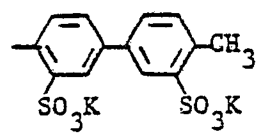 |
| 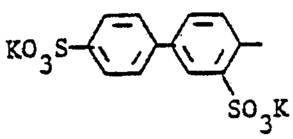 | 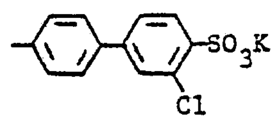 |
| 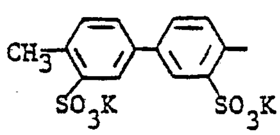 | 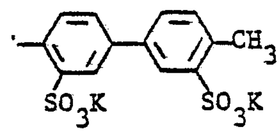 |

| A | A' |
| --- | --- |
| 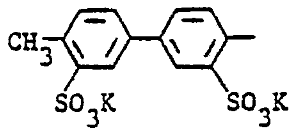 | 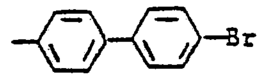 |
| 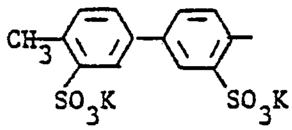 | 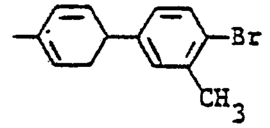 |
| 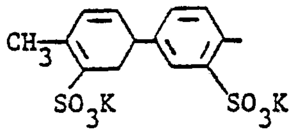 | 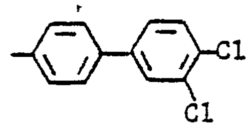 |
| 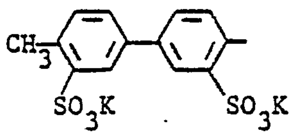 | 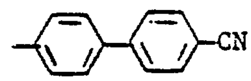 |
| 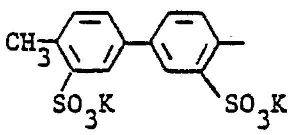 | 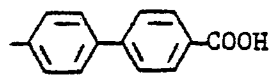 |
| 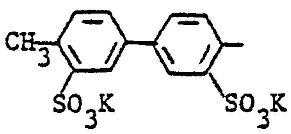 | 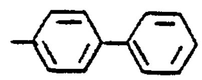 |
| 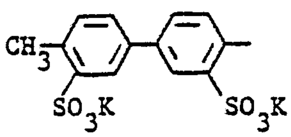 | 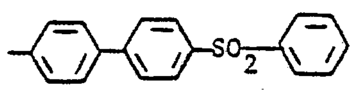 |
| 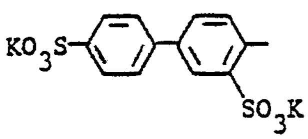 | 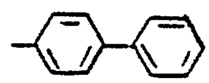 |
| 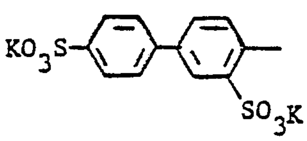 | 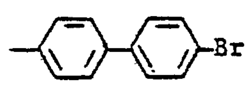 |
| 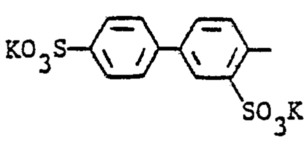 | 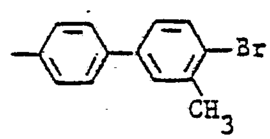 |

| A | A' |
| --- | --- |
| 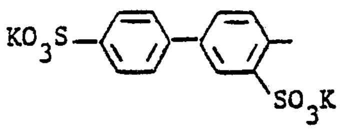 | 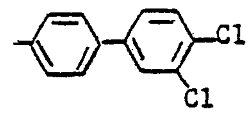 |
| 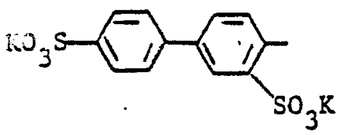 | 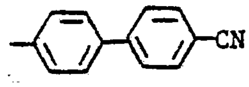 |
| 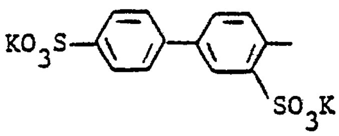 | 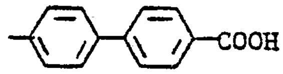 |
| 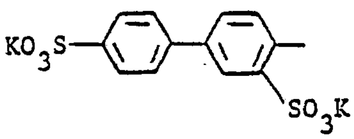 | 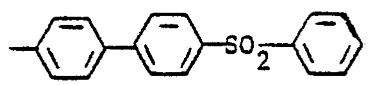 |
| 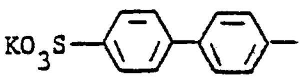 | 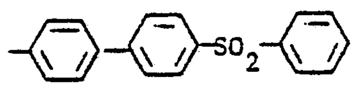 |

Patentansprüche:

1. Diphenylderivate der Formel

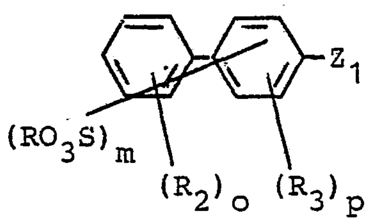

(I)

in der

R für Wasserstoff, ein salzbildendes Kation, einen Alkylrest mit 1 bis 8 Kohlenstoffatomen oder einen gegebenenfalls durch nicht chromophore Gruppen substituierten Aralkylrest,

$R_2$, $R_3$ unabhängig voneinander für Wasserstoff, Alkyl, Trifluormethyl, Alkoxy, Aralkoxy, Alkenyloxy, Halogen, die Carboxyl-, Cyan-, Alkylsulfon-, Arylsulfon-, Carbonamid-, Sulfonamid- oder die Carbonsäureestergruppe stehen,

m 1 oder 2 bedeutet,

o, p unabhängig voneinander für 0, 1 oder 2 stehen und

$Z_1$ die Aldehydgruppe oder einen Rest der Formeln

O O O $R_6$

$-CH_2-\overset{\|}{P}-OR_6$, $-CH_2-\overset{\|}{P}-OR_6$, $-CH_2-\overset{\|}{P}-R_6$ oder $-CH=P-R_6$

$OR_6$ $OR_6$ $R_6$ $R_6$

mit $R_6$ = $C_1$-$C_6$-Alkyl, Aryl, Aralkyl, Cycloalkyl, bezeichnet.

2. Biphenyl-4-aldehyd-3-sulfonsäure.

3. Biphenyl-4-aldehyd-3,4'-disulfonsäure.